# EUROPEAN PATENT SPECIFICATION

(11) **EP 3 852 629 B1**
(45) Date of publication and mention of the grant of the patent: **28.12.2022**
(21) Application number: 19828853.2
(22) Date of filing: 23.09.2019
(51) Int. Cl.: A61B 5/15, A61B 5/153, A61B 5/145, A61M 25/00

(54) **DEVICE FOR COLLECTING SPECIMEN IN A FLUID**
VORRICHTUNG ZUM SAMMELN VON PROBEN IN EINEM FLUID
DISPOSITIF DE PRÉLÈVEMENT D'UN ÉCHANTILLON DANS UN LIQUIDE

(30) Priority: 21.09.2018 NL 1043006
(43) Date of publication of application: 28.07.2021
(73) Proprietor: Idris Oncology B.V., 2333 CG Leiden (NL)
(72) Inventor: WIEGMAN, Peter Immanuël, 2311 HL LEIDEN (NL); MULDER, Hans Peter, 2315 BD LEIDEN (NL)
(74) Representative: EP&C
(86) International application number: PCT/NL2019/050637
(87) International publication number: WO 2020/060414

(56) References cited:
- EP-A1- 2 344 021
- WO-A1-2007/050718
- WO-A1-2009/021172
- WO-A1-2009/120239
- US-A- 5 782 759
- US-A1- 2016 101 262

## Description

### TECHNICAL FIELD

The various aspects and embodiments thereof relate to a device for collecting specimens from a fluid flow, including a blood flow of a living being and a human being in particular, in a vessel.

### BACKGROUND

Certain specimens in a body may provide an indication for a particular pathological disorder. If the location of the disorder is unknown, collection of such specimens is difficult. Some specimens may be taken along in the blood flow or the flow of other fluids in the body. As all blood passes the heart, the specimen may be collected in one of the large vessels connected to the heart or another vessel.

WO 2010/145824 discloses a detection device comprising a functional surface populated with detection receptors. The device has a three-dimensional structure of the functional surface. The structure has mutually facing functional sections which form clearances that can be penetrated by a sample fluid. The clearances are shaped as canals extending along the length of the device.

WO2006/116019 discloses endovascular brushes that include a brush segment at the distal end and a mandrel segment extending from the brush segment to the proximal end of the endovascular brush. The brush segment includes at least one brush element, such as a fibre, bristle, loop, ridge, or corrugation, configured to provide a space or spaced for retaining cells biopsied with the endovascular brush and to reduce the thrombogenicity of the endovascular brush, which allows a user to safely obtain therewith endovascular tissue from a targeted vascular segment *in-*vivo. The brush segment at the distal end of the endovascular brush includes at least one brush element, configured to retain biopsied cells on the brush. Hence, WO2006/116019 is aimed at retaining biopsied cells that have been dislodged from the vessel wall by an endovascular device in a biopsy procedure. Accordingly, the brush elements are configured to retain biopsied cells in the presence of the moving fluid.

EP2344021A1 discloses a device for detecting analytes, comprising a polymer fibre and capturing molecules, wherein the capturing molecules bind to an analyte and/or a linker molecule. The fibre-based device of EP2344021A1 is said to not only detect rare bioanalytes inside living organisms or in *in-vitro* samples, but which detects and catches such analytes in a highly efficient way and preserves or protects them until they can be *ex-situ* analysed in detail. EP2344021A1 discloses turbulator structures introduced into the fibre surface in order to enhance the analyte-surface interaction in a fluidic environment, as they are of a shape that locally enhances the effective surface area of the fibre. The turbulator structures may collect and protect the immobilised analytes during and after the collection process as it causes turbulence which attracts analytes towards the fibre surface.

These documents do not address the problem of reducing the velocity of the flow of the liquid when specimens are to be collected directly from that rapidly flowing liquid, which problem may for instance be very relevant when specimens are to be collected in high-velocity blood flows such as those occurring in vessels close to the heart.

### SUMMARY

It is an object of the invention to provide a device that provides a structure that provides improved collection of a specimen.

A first aspect of the invention provides a device for collecting a specimen in a fluid flowing through a vessel as claimed in claim 1. The device comprises an elongated body and a plurality of substantially continuous barriers, radially extending relative to the longitudinal axis from a surface of the body and provided along at least a part of the length of the body.

With the barriers provided radially extending from the device body along the length of the body, for example substantially perpendicularly to the longitudinal axis of the device body, the velocity of the flow of the liquid in which the specimens are to be collected is significantly reduced. Such reduction of flow velocity improves the odds of a specimen being retained by the device for collection.

In an embodiment, the barriers are shaped as directional receptacles having an opening directed to a distal end of the device body. Bodily flows, like the blood flow, have a pulsating nature due to the pumping activity of the heart. With the barriers shaped as directional receptacles having an opening directed towards a particular end of the device, specimens are driven in the receptacles at a particular phase of a heartbeat period. That is in particular the case if during that phase the flow of the blood is in the direction of the opening of the receptacle. During another phase, if the blood flow is directed away from the opening, the specimens are retained in the receptacle or at least the odds of the specimens leaving the receptacle under influence of the reversed blood flow are reduced.

In a further embodiment, the barrier is shaped to create a local vortex in the direct vicinity of the barrier if the directional flow is from the proximal end of the body to an opposite distal end of the body. Such barriers are relatively convenient to manufacture. With the flow recirculating in the receptacle, the specimens remain in the receptacle for an extended period.

In another embodiment, the barrier is shaped to create a local vortex in the direct vicinity of the barrier if the directional flow is in the opposite direction. In again another embodiment, the barrier is shaped to create a local vortex in the direct vicinity of the barrier if the directional flow is in both directions, substantially parallel to the length of the device.

In an embodiment, the barriers comprise concentric rings. The rings may be concentric with respect to each other and/or with respect to a longitudinal axis of the device. In alternative embodiments, the rings may be non-concentric with respect to each other and/or with respect to a longitudinal axis of the device.

In an embodiment, the thickness of the rings decreases from the body towards an outer perimeter of the rings distal to the device body.

The barriers may also have other shapes, wherein the radial, longitudinal and tangential dimensions may change along the circumference of the barriers. For example, the barriers may have a polygonal exterior shape with changing thickness and/or radial and/or longitudinal extent with respect to the longitudinal axis of the device along the circumference of the barrier. The shape of the barriers may be irregular along the circumference, such as an undulating radius or undulating longitudinal extent.

In yet another embodiment, the rings are tilted to the proximal end of the device body. This provides a simple yet effective receptacle.

In an embodiment, an outer part of the ring is tilted relative to the normal of the body under a first angle that is larger than a second angle under which an inner part of the ring is tilted relative to the normal.

In yet a further embodiment, adjacent barriers form a cavity. A cavity provides a space for effective retention of specimens.

In an embodiment, a first end of a first barrier proximal to the cavity, the first end defining a proximal part of the cavity, extends further from the body than a second end of a second barrier distal to the cavity, the second end defining a distal end of the cavity or vice versa.

In an embodiment, the cavity is shaped as a ring having a substantially circular cross-section.

The cavity may also have any other suitable shape extending around the elongate body. As the cavity is formed between two adjacent barriers, the shape of the cavity is defined by the shape of the barriers. With changing shape of the barriers along the circumference of the respective barrier, the shape of the cavity, for example the width or depth of the cavity, may also change.

In longitudinal direction of the device, different types or shapes of barriers may be used in a single device. These different types or shapes of barriers may be used to form differently shaped cavities along the longitudinal direction of the device to influence the flow characteristics of a fluid flow flowing along the barriers in order to improve specimen collection by the device. For example, different types of cavities may for example be designed for different fluid flow speeds or directions along the device.

A second aspect provides a kit of parts comprising the device according to the first aspect, a guide wire connected to the device and a tube through which the guide wire is provided, the tube being arranged to receive the device.

A third aspect (not claimed) provides the use of the device of the first aspect for collecting specimen in a vessel, for example a blood vessel of a body of a living being and human being in particular.

The vessel may be in vivo or ex vivo.

In the third aspect the device may be used for collecting specimen in the superior vena cava (use not claimed).

### BRIEF DESCRIPTION OF THE DRAWINGS

The various aspects and embodiments thereof will now be elucidated in conjunction with Figures. In the Figures:
Figure 1: shows a schematic isometric view of an embodiment of the device;
Figure 2: shows a part of a cross-section along the length of an embodiment of the device;
Figure 3 A: shows a diagram schematically depicting flow velocity during a cardiac cycle;
Figure 3 B: shows a specimen in a vessel under influence of the pulsating nature of the flow of a liquid in which the specimen is provided;
Figure 3 C: shows a specimen in a vessel with an embodiment of the device;
Figure 3 D: shows a specimen in a vessel with another embodiment of the device or under alternate flow conditions;
Figure 4 A: shows a part of a cross-section along the length of another embodiment of the device;
Figure 4 B: shows a specimen traveling along the perimeter of this embodiment of the device under influence of a pulsating fluid flow;
Figure 4 C: shows a specimen traveling along another embodiment of the device or under influence of a differently pulsating fluid flow;
Figure 5: shows the device being retracted in a tube for preservation of the specimen;
Figure 6: shows a system for collecting a specimen in a vessel of a body, preferably a blood vessel like a vein in a human body;
Figure 7 A: shows another device as another embodiment of the first aspect; and
Figure 7 B shows a cross-section of the device depicted by Figure 7 A.

### DETAILED DESCRIPTION

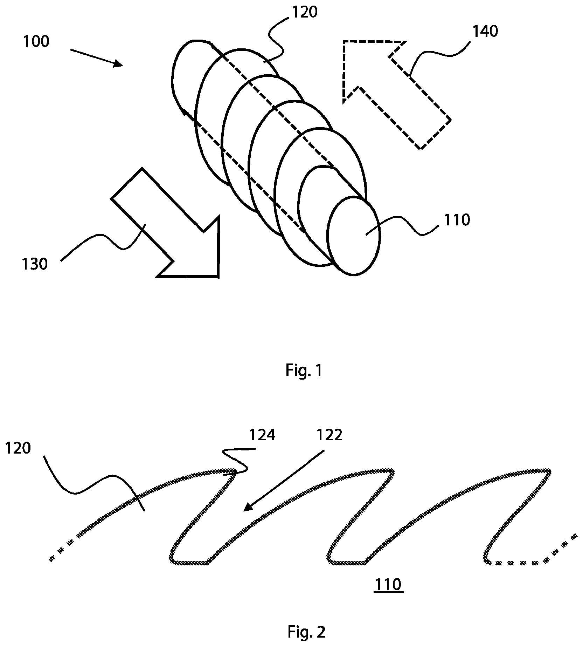

Figure 1 shows a device 100 for collecting specimen from a fluid flow, including a blood flow of a living being and a human being in particular in a vessel. The specimen may be a biological specimen, a chemical specimen, other, or a combination thereof. Biological specimens include, but are not limited to, (poly)saccharides, (poly)nucleotides, (poly)peptides, and combinations thereof, such as nucleated or non-nucleated cells or tissues or parts thereof.

The device 100 comprises a device body 110. Around the device body, concentric rings 120 are provided as substantially continuous barriers extending radially from the device body 110. In this embodiment, the planes of the rings 120 are provided substantially perpendicular to the longitudinal axis of the body 110. In another embodiment, the rings 120 may be placed under an angle less than ninety degrees relative to the longitudinal axis of the device body 110.

Radially extending means that each barrier is provided at one particular point along the centre axis and does not extend from a first position along the centre axis to a second point along the centre axis at a distance from the first point. The position of the barrier at which the barrier is provided on the device body may vary, as the barrier extends along the circumference of the outer wall of the body 110, the barrier ends at substantially the same position as where it begins, i.e. it does not spiral along the outer wall of the device body 110. The average directional vector of a line along the connection between the barrier and the device body is substantially zero in the direction of the longitudinal axis, over the full length of that connection.

In Figure 1, a first arrow 130 and a second arrow 140 indicate a blood flow in a vessel in which the device 100 may be inserted for collecting specimen. The concentric rings 120 are provided as barriers that are positioned on the body 110 such that the barriers are substantially perpendicular to the flow.

Furthermore, the barriers are provided substantially continuously around the body. With substantially continuous is meant that the barriers circumferentially enclose the body for at least 60% - for example having the shape of a three-quarter circle - more preferably 70%, 75%, 80%, 85% or 90% and most preferably 100%.

Figure 2 shows a cross-section of the device 100 with the barriers 120 having a different shape. The barriers 120 shown by Figure 2 circumferentially enclose the body 110. Furthermore, the barriers 120 as shown by Figure 2 are tilted towards a distal end of the body 110. In this embodiment, the barriers 120 have the cross-section of a shark fin or dolphin fin. The barriers are thicker near the body 110 and the thickness of the barriers 120 decreases as the barriers 120 extend from the body.

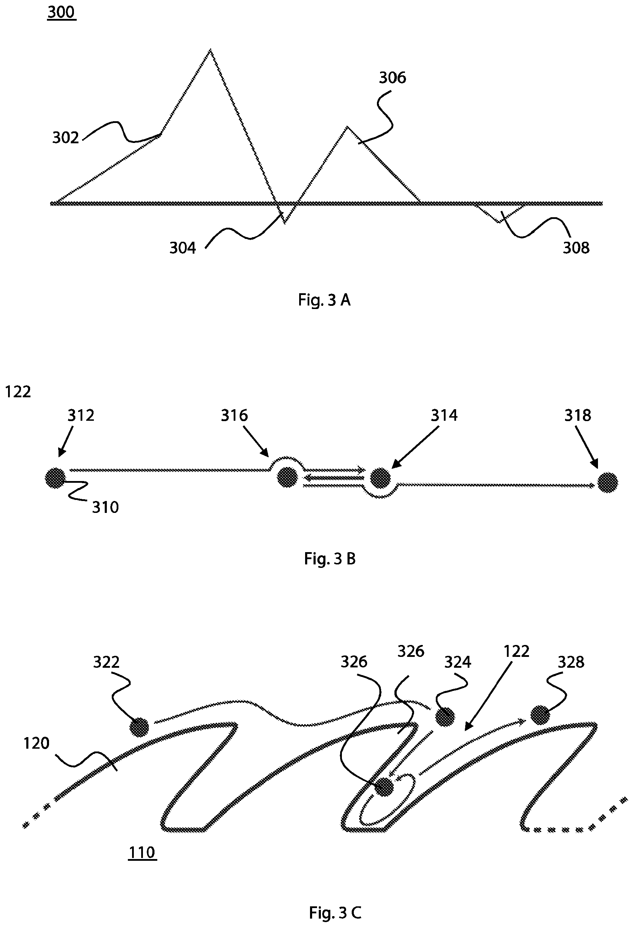

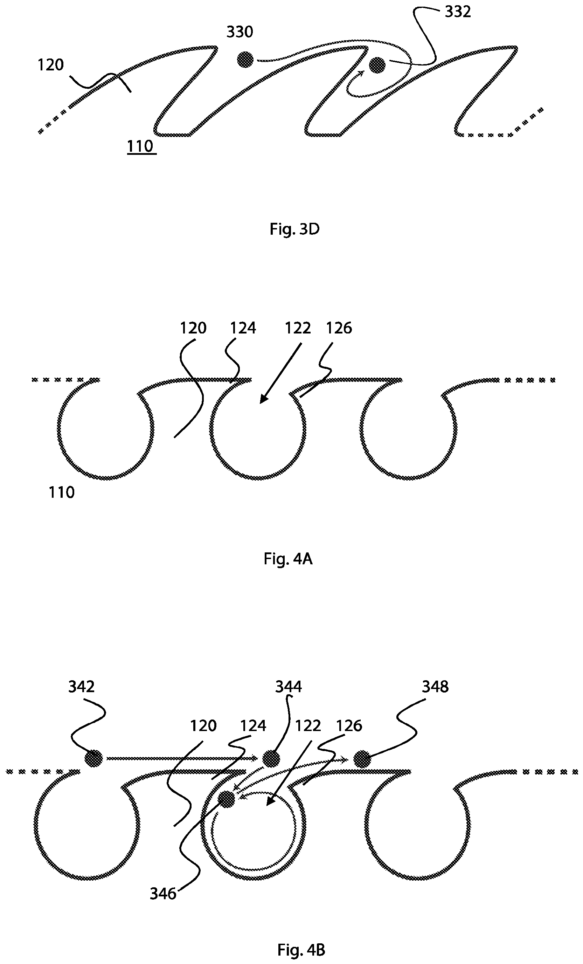

This shaping of the barriers 120 constitutes on a per-barrier basis a receptacle 122 that opens towards the distal side of the receptacle 122 and at the outer perimeter of the device 100. Hence, the receptacles 122 are provided as directional receptacles 122 in the sense that the opening of the receptacles 122 is directed towards the distal end of the device, rather than straight outward from the body 110. This will be elucidated further in conjunction with Figure 3 A, Figure 3 B, Figure 3 C and Figure 3 D. In another embodiment, the receptacles are open towards the proximal side of the device 100.

In preferred embodiments of aspects of this invention, the barriers are shaped as directional receptacles having an opening directed to either a distal or proximal end of the device body. Whereas prior art devices aim to increase surface or contact area between liquid and device surface or increase turbulence of liquid at the device surface in order to improve collection of analytes, the present invention in preferred aspects uses receptacles having an opening in a specific direction relative to the longitudinal axis of the device. Such directional receptacles find particular beneficial application in blood flow sampling in living organisms because hemodynamics in such situations is characterized by its pulsatility, i.e., the difference in the systolic and diastolic velocities in the circulation of the blood. Preferred embodiments of aspects of this invention wherein the barriers are shaped as directional receptacles are in particular, but not exclusively, beneficial when considering the hemodynamics in or near the superior vena cava near the heart as depicted in Figure 3 A.

Figure 3 A shows a graph 300 depicting schematically the speed of blood through the superior vena cava near the heart. The vertical axis indicates flow speed. The horizontal axis, which crosses the vertical axis at a value of zero, indicates time. A first graph part 302 indicates an increasing and decreasing speed of the blood flow towards the heart. This forward flow of blood is generated during the atrial diastole and ventricular systole. A second graph part 304 indicates a brief flow of blood away from the heart, which may be generated during the transition from ventricular systole to diastole. Subsequently, a third graph part 306 indicates again an increasing and decreasing blood flow towards the heart during the ventricular diastole. After a short period of no blood flow, a fourth graph part 308 indicates a brief flow of blood away from the heart by virtue of the atrial systole. After this phase the flow repeats this pattern.

Due to the atrial and ventricular diastole and systole, the blood in the superior vena cava exhibits a pulsating nature. Specimens in the blood flow along with the blood and exhibit a pulsating movement as well. This is shown in Figure 3 B. Figure 3 B shows a white blood cell 310 as a biological specimen in the blood in the superior vena cava. During the ventricular diastole phase, the white blood cell moves along with the average flow direction of the blood in the vessel, from a first position 312 to a second position 314. During the atrial systole, the white blood cell moves backward due to the average flow in the vessel, from the second position 314 to the third position 316. Subsequently, during the atrial diastole and ventricular systole phase, the white blood cell 310 moves from the third position 316 to the fourth position 318 in the vessel.

Figure 3 B shows the unaffected flow in the superior vena cava. In another vein or artery, the pulsating displacement may differ greatly, e.g. being less pulsatile in peripheral veins, or having a much higher peak, but little backflow in the pulmonary artery. Furthermore, Figure 3 B shows the movement of the white blood cell 310 in an unobstructed blood conduit, i.e. the vessel, without any artefacts in the way.

Figure 3 C shows the flow of the white blood cell in a blood vessel and the superior vena cava in particular with the device 100 provided in the vessel. The device 100 is provided in the vessel such that the length of the device 100 is substantially parallel to the average blood flow direction in the vessel. In the embodiment shown by Figure 3 C, the device 100 is placed in the vessel such that the distal end of the device 100 is provided downstream of the blood flow and the opposite proximal end is provided upstream relative to the distal end.

By virtue of this placement of the device 100 and the tilting of the barriers 120, the white blood cell flows along the opening of the receptacle 122 during the ventricular diastole, from a first position 322 to a second position 324. With the white blood cell 310 being in the proximity of the directional opening of the receptacle 122 at the end of the ventricular diastole, the white blood cell 310 may flow in the receptacle 122 during the atrial systole to a third position 326.

In the receptacle 122, the white blood cell 310 is under reduced influence of the general blood flow in the vessel. Therefore, depending on the shape of the receptacle 122, the blood flow in the receptacle 122 may be lower than in the vessel without obstructions. Moreover, due to the general blood flow and the shape of the shark fin-shaped cross-section of the barrier 120, a vortex may occur in the blood flow in the receptacle 122. Due to the vortex or other non-linear and potentially turbulent flow of blood in the receptacle 122 and the preferably lower speed of the blood flow in the receptacle 122, the white blood cell 310 may remain in the receptacle 122 for an extended period, rather than leaving the receptacle 122 directly under influence of the atrial diastole and ventricular systole, moving to the fourth position 328.

While the white blood cell 310 resides in the receptacle 122 and with the blood flow in the receptacle 122 being different and preferably lower than in the general space of the vessel, the white blood cell 310 may bind to the surface of the device 100 and to the surface of the barriers 120, the surface of the body 110 or both in particular.

In the embodiments shown thus far the device 100 has been shown as inserted in a vessel such that a receptacle can collect a specimen during the atrial systolic phase. In another embodiment, the device 100 is inserted in the vessel such that receptacle 122 can collect a specimen during other phases, such as the atrial diastolic and ventricular systolic phase.

Figure 3 D shows an alternate embodiment or an alternate flow condition where the specimen 310 is carried closely over the surface of the barrier 120. When the flow reaches the apex of the barrier, flow separation may be caused. This may result in a turbulent or vortical flow in the receptacle 122. Due to this turbulent or vortical flow, the specimen 310 is carried from its initial position 330 along with the separating flow and into the receptacle 122 to its second position 332, where it may subsequently behave in similar fashion as described in Figure 3 C.

Figure 4 A shows another embodiment of the device 100 with differently shaped receptacles 122. In the embodiment shown by Figure 4 A, the barriers 120 extend substantially perpendicularly from the body 110. The barriers 120 are shaped concavely at both sides of the concentric rings such that the receptacle 122 has a substantially circular cross-section. The extremities of the barriers are shaped asymmetrically such that a first overhang 124 extends further over the receptacle 122 than a second overhang 126. Furthermore, the first overhang 124 extends further from the body 110 than the second overhang 126. This results in the opening of the receptacle 122 being directed towards a distal end of the device 100 and in that way, in the receptacle 122 being directional.

Figure 4 B shows the device as shown by Figure 4 A, with a white blood cell 310 at various positions along the device 100 during various phases of a heartbeat. Due to the different shapes of the first overhang 124 and the second overhang 126, the white blood cell is enabled to move over the top side of the first overhang 124 from a first position 342 to a second position 344 during ventricular diastole. Then it can move under the lower side of the first overhang 124 into the receptacle 122 at a third position 346 during the atrial systolic phase. Ultimately, the white blood cell may leave the receptacle 122 and move to a fourth position 348 or stay within the receptacle.

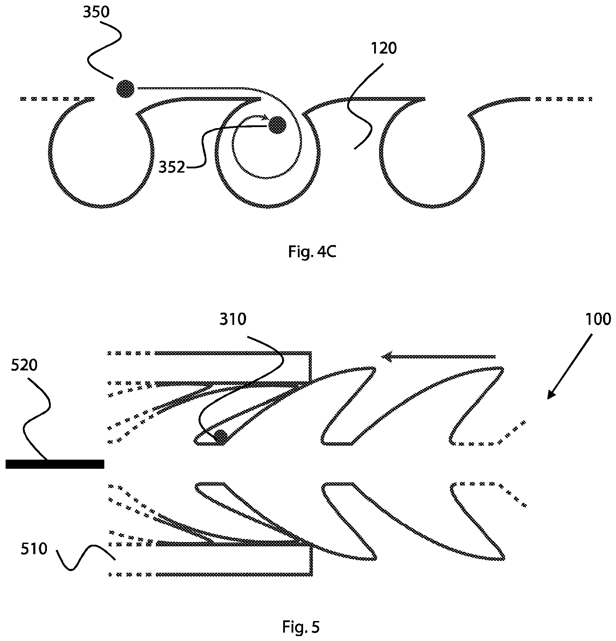

Figure 4 C shows an alternate embodiment or an alternate flow condition where the flow into the receptacle 122 is caused by flow separation, similar to the description of Figure 3D. Flow reaching the apex of the overhang 124 is unable to follow the contour, and instead separates into turbulent or vertical flow into the receptacle 122, carrying the specimen 310 from its initial position 350 into the receptacle 122, in position 352.

With the specimen collected in one or more receptacles 122, the device 100 may be withdrawn from the vessel for further analysis of the specimen, as shown in Figure 5. To this purpose, the device 100 is connected to a string like a rod or a wire 520. The wire 520 may be provided in a single metal or a metal alloy, including, but not limited to surgical steel, titanium or a titanium rich alloy, other, or a combination thereof. Alternatively, the wire may be comprised of an organic polymer, including, but not limited to poly ether ketone (PEEK), PE, another, or a combination thereof. In yet another embodiment, the wire 520 comprises an optical fibre that may be used for optical inspection.

The wire 520 is provided in a tube 510 for pulling the device 100 into the tube. In a preferred embodiment, the inner diameter of the tube 510 is slightly smaller than the outer diameter of the device 100. With the barriers 120 preferably having a flexible and more preferably a resilient nature, extremities of the barriers 120 are pushed towards surfaces of adjacent barriers. This is preferably done such that the receptacles 122 are closed off. This aids in preservation of the specimen in the receptacles 122.

The device preferably predominantly comprises a polymer material to provide resilience. The polymer may be a cross-linked polymer, polyurethane(PU), poly ether ketone (PEEK), polyethylene, another polymer, either organic or not, other material or a combination thereof.

Within the body 110 of the device 100, a wire may be provided to move the device 100 along a vein and in the tube 510. Such a wire may also provide rigidity. Such a wire may be comprised of steel, a polymeric material and an organic polymeric material in particular, one or more carbon fibres, other, or a combination thereof. Alternatively such a wire may be the same wire as 520 that is extended through the device body.

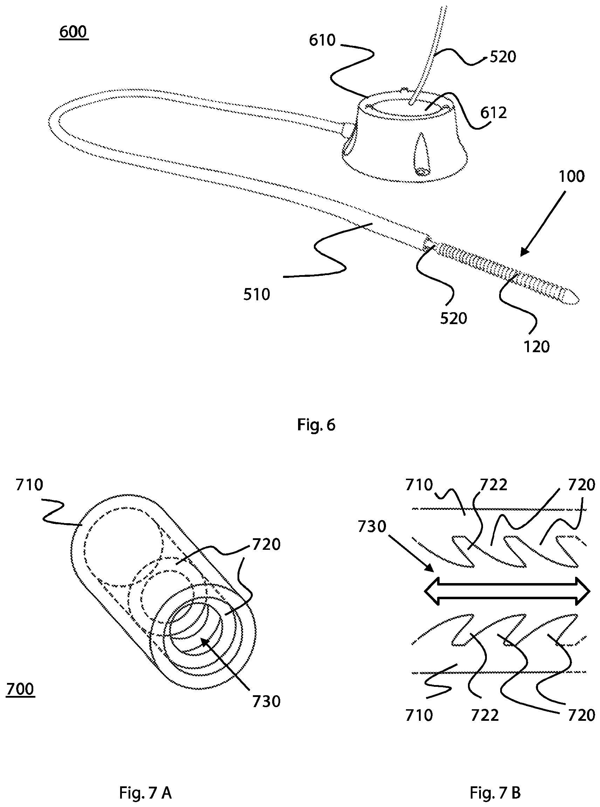

Figure 6 shows a system 600 for collecting a specimen in a vessel of a body, preferably a blood vessel such as a vein or artery in a human body. The system comprises the device 100, the wire 520 and the tube 510. The tube 510 is connected to the inner reservoir 610. The reservoir 610 is preferably implanted in the body of the subject on which the device 100 is used for collecting specimen. Human beings may be provided with the internal reservoir 610 within their bodies for intravenous distribution of drugs. To this purpose, the reservoir 610 is connected to a vessel via the tube 510.

The wire 520 protrudes through a top membrane 612 of the reservoir 610 and is led through the tube 510 to the device 100. As indicated in Figure 6, the proximal end of the device 100 is connected to the wire 520, with the barriers tilted towards the distal end. In another embodiment, the barriers are tilted towards the proximal end.

Figure 7 A shows an isometric schematic view of a device 700 as another embodiment of the first aspect. The device 700 comprises a hollow device body 710 having a conduit 730 provided in the device 700, along a longitudinal axis of the device body 710. From the inner wall of the device body 710, barriers 720 radially extend towards the longitudinal (centre) axis of the device 700. The barriers 720 may be provided substantially perpendicular to the longitudinal axis or may be tilted relative to the longitudinal axis.

The latter option is shown by Figure 7 B, which shows a cross-section of the device 700 along the longitudinal axis of the device 700. Figure 7 B shows the barriers 720 having a shark-fin shaped cross-section, which provides a directional receptacle 722. Alternatively, the barriers 720 may be shaped as shown by Figure 4 A or otherwise for forming a directional receptacle.

The device 700 may be provided as a tube, with the device body being predominantly shaped as a tube. In another embodiment, the device body 700 may be significantly larger, with multiple conduits 730 provided substantially parallel to one another.

Thus far, the barriers radially extending from the device body have been discussed as having a substantially circular outer perimeter. In other embodiments, the perimeter of the core, the outer perimeter of the barriers, or both, whether the barriers extend inwardly or outwardly, may have an elliptical, oval, square, triangular or any other shape, either of a polygon, a curve, other, or a combination thereof.

In an embodiment, different types or shapes of barriers may be used in a single device. These different types or shapes of barriers may be applied to influence the flow characteristics of a fluid flow flowing along the barriers in order to improve specimen collection by the device.

The embodiments discussed thus far have been discussed in conjunction with use of the device for collecting specimen in vivo, i.e. for collecting specimen in a vessel and in particular a blood vessel of a body of a living being and human being in particular. The device may, with similar shapes, also be used for collecting specimen in blood or another bodily fluid and bodily liquid in particular ex vivo, in a vessel outside a body. This may be in a conduit connected to the human vascular system, for example in a conduit of a renal dialysis device connected to a body. Alternatively, the device may be used in a conduit or other vessel not connected to a human body. But the device may also be used for collecting specimen in other flows, like a flow of crude oil in a pipeline, a flow of water in a water supply system, a gas flow in a natural gas distribution system or another vessel carrying a fluid carrying specimen that may have to be collected for further analysis or for removal of these specimen per se.

In the description above, it will be understood that when an element such as layer, region or substrate is referred to as being "on" or "onto" another element, the element is either directly on the other element, or intervening elements may also be present. Also, it will be understood that the values given in the description above, are given by way of example and that other values may be possible and/or may be strived for.

Furthermore, the invention may also be embodied with less components than provided in the embodiments described here, wherein one component carries out multiple functions. Just as well may the invention be embodied using more elements than depicted in the Figures, wherein functions carried out by one component in the embodiment provided are distributed over multiple components.

It is to be noted that the figures are only schematic representations of embodiments of the invention that are given by way of non-limiting examples. For the purpose of clarity and a concise description, features are described herein as part of the same or separate embodiments, however, it will be appreciated that the scope of the invention may include embodiments having combinations of all or some of the features described. The word 'comprising' does not exclude the presence of other features or steps than those listed in a claim. Furthermore, the words 'a' and 'an' shall not be construed as limited to 'only one', but instead are used to mean 'at least one', and do not exclude a plurality.

A person skilled in the art will readily appreciate that various parameters and values thereof disclosed in the description may be modified and that various embodiments disclosed and/or claimed may be combined without departing from the scope of the invention.

It is stipulated that the reference signs in the claims do not limit the scope of the claims, but are merely inserted to enhance the legibility of the claims.

## Claims

1. Device (100, 700) for collecting a specimen (310) in a fluid in a vessel, the device comprising an elongated body (110; 710) and a plurality of substantially continuous barriers (120; 720), radially extending from the body relative to the longitudinal axis from a surface of the body and provided along at least a part of the length of the body.

2. Device (100, 700) according to claim 1, wherein the barriers (120; 170) are shaped as directional receptacles (122) having an opening directed to a distal end of the device body or directed to a proximal end.

3. Device (100, 700) according to claim 1 or claim 2, wherein the barrier (120; 170) is shaped to create a local vortex in the direct vicinity of the barrier if the directional flow is from the proximal end of the body (110; 710) to an opposite distal end of the body or vice versa.

4. Device (100, 700) according to claim 3 to the extent dependent on claim 2, wherein the receptacle (122) is shaped to create the vortex in the receptacle.

5. Device (100, 700) according to any of the preceding claims, wherein the barriers (120; 170) comprise concentric rings.

6. Device (100, 700) according to claim 5, wherein the thickness of the rings decreases from the body (110; 710) towards an outer perimeter of the rings distal to the device body.

7. Device (100, 700) according to claim 5 or 6, wherein the rings are tilted to a distal end of the device body (110; 710) or towards a proximal end.

8. Device (100, 700) according to claim 7, wherein an outer part of the ring is tilted relative to the normal of the body (110; 710) under a first angle that is larger than a second angle under which an inner part of the ring is tilted relative to the normal.

9. Device (100, 700) according to any of the preceding claims, wherein adjacent barriers (110; 710) form a cavity.

10. Device (100, 700) according to claim 9, wherein a first end of a first barrier (120; 170) proximal to the cavity, the first end defining a proximal part of the cavity, extends further from the body (110; 710) than a second end of a second barrier distal to the cavity, the second end defining a distal end of the cavity or vice versa.

11. Device (100, 700) according to claim 9 or 10, wherein the cavity is shaped as a ring having a substantially circular cross-section.

12. Device (100, 700) according to any of the preceding claims,
wherein the device predominantly comprises one or more polymer materials selected from the group:
- polyether ether ketone;
- polyethylene;
- polyurethane; and
- polysiloxane

13. Device (100, 700) according to any of the preceding claims, further comprising a wire, preferably comprising a metal, a metal alloy, an organic polymer, an optical fibre, another structurally supporting material, or a combination thereof, provided substantially in the centre of the body.

14. Device (100, 700) according to any of the preceding claims,
wherein the device body (710) comprises a conduit (730) through which a fluid flow may be provided and the barriers (720) extend from an inner wall surface of the conduit towards the longitudinal axis of the device.

15. Device (100, 700) according to any of the preceding claims, wherein the barriers (120) extend from an outer wall surface of the body.

16. Kit of parts comprising:
- the device (100, 700) according to any of the preceding claims;
- a guide wire (520) connected to the device; and
- a tube (510) through which the guide wire is provide, the tube being arranged to receive the device.

## Patentansprüche

1. Vorrichtung (100, 700) zum Sammeln einer Probe (310) in einem Fluid in einem Gefäß, wobei die Vorrichtung einen länglichen Körper (110; 710) und eine Vielzahl von im Wesentlichen kontinuierlichen Barrieren (120; 720) umfasst, die sich radial von dem Körper relativ zu der Längsachse von einer Oberfläche des Körpers aus erstrecken und entlang mindestens eines Teils der Länge des Körpers vorgesehen sind.

2. Vorrichtung (100, 700) nach Anspruch 1, wobei die Barrieren (120; 170) als gerichtete Aufnahmen (122) mit einer zu einem distalen Ende des Vorrichtungskörpers oder zu einem proximalen Ende gerichteten Öffnung ausgebildet sind.

3. Vorrichtung (100, 700) nach Anspruch 1 oder Anspruch 2, wobei die Barriere (120; 170) so geformt ist, dass sie einen lokalen Wirbel in der unmittelbaren Umgebung der Barriere erzeugt, wenn die Strömungsrichtung vom proximalen Ende des Körpers (110; 710) zu einem entgegengesetzten distalen Ende des Körpers oder umgekehrt verläuft.

4. Vorrichtung (100, 700) nach Anspruch 3 in dem von Anspruch 2 abhängigen Umfang, wobei die Aufnahme (122) so geformt ist, dass der Wirbel in der Aufnahme erzeugt wird.

5. Vorrichtung (100, 700) nach einem der vorhergehenden Ansprüche, wobei die Barrieren (120; 170) aus konzentrischen Ringen bestehen.

6. Vorrichtung (100, 700) nach Anspruch 5, wobei die Dicke der Ringe vom Körper (110; 710) zu einem äußeren Umfang der Ringe distal zum Vorrichtungskörper hin abnimmt.

7. Vorrichtung (100, 700) nach Anspruch 5 oder 6, wobei die Ringe zu einem distalen Ende des Vorrichtungskörpers (110; 710) oder zu einem proximalen Ende hin geneigt sind.

8. Vorrichtung (100, 700) nach Anspruch 7, wobei ein äußerer Teil des Rings relativ zur Normalen des Körpers (110; 710) unter einem ersten Winkel geneigt ist, der größer ist als ein zweiter Winkel, unter dem ein innerer Teil des Rings relativ zur Normalen geneigt ist.

9. Vorrichtung (100, 700) nach einem der vorhergehenden Ansprüche, wobei benachbarte Barrieren (110; 710) einen Hohlraum bilden.

10. Vorrichtung (100, 700) nach Anspruch 9, wobei ein erstes Ende einer ersten Barriere (120; 170) proximal zu dem Hohlraum, wobei das erste Ende einen proximalen Teil des Hohlraums definiert, sich weiter von dem Körper (110; 710) erstreckt als ein zweites Ende einer zweiten Barriere distal zu dem Hohlraum, wobei das zweite Ende ein distales Ende des Hohlraums definiert oder umgekehrt.

11. Vorrichtung (100, 700) nach Anspruch 9 oder 10, wobei der Hohlraum als Ring mit einem im Wesentlichen kreisförmigen Querschnitt geformt ist.

12. Vorrichtung (100, 700) nach einem der vorhergehenden Ansprüche, wobei die Vorrichtung überwiegend ein oder mehrere Polymermaterialien umfasst, ausgewählt aus der Gruppe:
- Polyetheretherketon;
- Polyethylen;
- Polyurethan; und
- Polysiloxan.

13. Vorrichtung (100, 700) nach einem der vorhergehenden Ansprüche, die weiter einen Draht umfasst, der vorzugsweise ein Metall, eine Metalllegierung, ein organisches Polymer, eine optische Faser, ein anderes strukturelles Trägermaterial oder eine Kombination davon umfasst und im Wesentlichen in der Mitte des Körpers vorgesehen ist.

14. Vorrichtung (100, 700) nach einem der vorhergehenden Ansprüche, wobei der Vorrichtungskörper (710) eine Leitung (730) umfasst, durch die ein Fluidstrom bereitgestellt werden kann, und die Barrieren (720) sich von einer Innenwandfläche der Leitung in Richtung der Längsachse der Vorrichtung erstrecken.

15. Vorrichtung (100, 700) nach einem der vorhergehenden Ansprüche, wobei sich die Barrieren (120) von einer Außenwandfläche des Körpers aus erstrecken.

16. Teilesatz bestehend aus:
- der Vorrichtung (100, 700) nach einem der vorhergehenden Ansprüche;
- einen Führungsdraht (520), der mit der Vorrichtung verbunden ist; und
- einem Rohr (510), durch das der Führungsdraht geführt ist, wobei das Rohr so angeordnet ist, dass es die Vorrichtung aufnehmen kann.

## Revendications

1. Dispositif (100, 700) pour prélever un échantillon (310) dans un fluide dans un récipient, le dispositif comprenant un corps allongé (110 ; 710) et une pluralité de barrières (120 ; 720) sensiblement continues, s'étendant radialement du corps par rapport à l'axe longitudinal à partir d'une surface du corps et prévues le long d'au moins une partie de la longueur du corps.

2. Dispositif (100, 700) selon la revendication 1, dans lequel les barrières (120 ; 720) sont formées comme des réceptacles directionnels (122) ayant une ouverture dirigée vers une extrémité distale du corps de dispositif ou dirigée vers une extrémité proximale.

3. Dispositif (100, 700) selon la revendication 1 ou la revendication 2, dans lequel la barrière (120 ; 170) est formée pour créer un tourbillon local dans la proximité directe de la barrière si le flux directionnel va de l'extrémité proximale du corps (110 ; 710) à une extrémité distale opposée du corps ou vice versa.

4. Dispositif (100, 700) selon la revendication 3 dans la mesure où elle dépend de la revendication 2, dans lequel le réceptacle (122) est formé pour créer le tourbillon dans le réceptacle.

5. Dispositif (100, 700) selon l'une quelconque des revendications précédentes, dans lequel les barrières (120 ; 170) comprennent des bagues concentriques.

6. Dispositif (100, 700) selon la revendication 5, dans lequel l'épaisseur des bagues diminue du corps (110 ; 710) vers un périmètre externe des bagues à distance du corps de dispositif.

7. Dispositif (100, 700) selon la revendication 5 ou 6, dans lequel les bagues sont inclinées vers une extrémité distale du corps de dispositif (110 ; 710) ou vers une extrémité proximale.

8. Dispositif (100, 700) selon la revendication 7, dans lequel une partie externe de la bague est inclinée par rapport à la normale du corps (110 ; 710) à un premier angle qui est supérieur à un second angle auquel une partie interne de la bague est inclinée par rapport à la normale.

9. Dispositif (100, 700) selon l'une quelconque des revendications précédentes, dans lequel les barrières (110 ; 710) adjacentes forment une cavité.

10. Dispositif (100, 700) selon la revendication 9, dans lequel une première extrémité d'une première barrière (120 ; 170) à proximité de la cavité, la première extrémité définissant une partie proximale de la cavité, s'étend davantage à partir du corps (110 ; 710) qu'une seconde extrémité d'une seconde barrière à distance de la cavité, la seconde extrémité définissant une extrémité distale de la cavité ou vice versa.

11. Dispositif (100, 700) selon la revendication 9 ou 10, dans lequel la cavité est formée comme une bague ayant une section transversale sensiblement circulaire.

12. Dispositif (100, 700) selon l'une quelconque des revendications précédentes, dans lequel le dispositif comprend, de manière prédominante, un ou plusieurs matériaux polymères sélectionnés dans le groupe comprenant :
le polyétheréthercétone ;
le polyéthylène ;
le polyuréthane ; et
le polysiloxane.

13. Dispositif (100, 700) selon l'une quelconque des revendications précédentes, comprenant en outre un fil, comprenant de préférence un métal, un alliage métallique, un polymère organique, une fibre optique, un autre matériau de support structurel ou leur combinaison, prévu sensiblement au centre du corps.

14. Dispositif (100, 700) selon l'une quelconque des revendications précédentes, dans lequel le corps de dispositif (710) comprend un conduit (730) à travers lequel un écoulement de fluide peut être prévu et les barrières (720) s'étendent à partir d'une surface de paroi interne du conduit vers l'axe longitudinal du dispositif.

15. Dispositif (100, 700) selon l'une quelconque des revendications précédentes, dans lequel les barrières (120) s'étendent à partir d'une surface de paroi externe du corps.

16. Kit de pièces comprenant :
le dispositif (100, 700) selon l'une quelconque des revendications précédentes ;
un fil-guide (520) raccordé au dispositif ; et
un tube (510) à travers lequel le fil-guide est prévu, le tube étant agencé pour recevoir le dispositif.
